# EUROPÄISCHE PATENTANMELDUNG

(11) **EP 0 848 932 A1**
(43) Veröffentlichungstag der Anmeldung: **24.06.1998**
(21) Anmeldenummer: 97121872.2
(22) Anmeldetag: 11.12.1997
(51) Int. Cl.: A61B 5/11, A61B 9/00

(54) **Gerät zum Untersuchen von elektrisch stimulierten Muskeln sowie Untersuchungsverfahren für Muskelgewebe**

(30) Priorität: 20.12.1996 DE 19653491
(71) Anmelder: Bavaria Patente und Lizenzen Verwertungsgesellschaft mbH, 01731 Kreischa (DE)
(72) Erfinder: Blümel, Georg, Doz.Dr.sc.nat., 04209 Leipzig (DE)
(74) Vertreter: von Samson-Himmelstjerna, Friedrich R., Dipl.-Phys.

(57) **Zusammenfassung**

Die Erfindung betrifft ein Gerät (UA) zum Untersuchen von mittels elektrischer Impulse (S) stimulierter Muskeln oder Muskelgruppen, bei dem die Signale der mechanischen Reizantwort der Muskeln über mindestens einen Meßwertwandler (M; TG), der mechanische Signale in elektrische Signale umwandelt und dessen Fühler hautextern auf den zu untersuchenden Muskeln aufliegt, an mindestens eine Darstellungs- und/oder Aufzeichnungsvorrichtung (O) weiterleitbar sind, wobei der Meßwertwandler (M; TG) so ausgebildet ist, daß der Verlauf (D) seiner elektrischen Ausgangssignale proportional der zeitlichen Ableitung (Differentiation) des Verlaufs seiner mechanischen Eingangssignale ist. Außerdem betrifft die Erfindung ein Untersuchungsverfahren zum Feststellen eines physiologischen Zustandes von Muskelgewebe mit den Schritten: Beaufschlagen des Muskelgewebes mit einem elektrischen Impuls; Aufnehmen eines Reizantwortsignal; und Messen der Latenzzeit, Dauer der aktiven Phase und/oder Dauer der Ausklingphase und/oder einer Kombination dieser Zeiten.

## Beschreibung

Die Erfindung betrifft ein Gerät zum Untersuchen von mittels elektrischer Impulse stimulierten Muskeln mit den im Gattungsbegriff des Anspruchs 1 angegebenen Merkmalen. Außerdem betrifft die Erfindung ein Untersuchungsverfahren zum Feststellen eines physiologischen Zustandes von Muskelgewebe.

Bei bekannten Geräten dieser Art ist der Meßwertwandler mit einem mechanischen Meßwertaufnehmer ausgestattet, der als Magnetfeldgeber/Hall-Sensor (DE 36 01 930 A1, PHYSIOMED), Dehnungsmeßstreifen (DE 39 39 790 C1, DRÄGERWERK), piezoelektrischer Film (WO 92/03974, AXON) oder Stethoskop/Mikrophon (DE 36 42 237 A1, MEYER) ausgebildet ist.

Bei allen diesen Geräten werden die mechanischen Signale im wesentlichen proportional in die entsprechenden elektrischen Signale umgewandelt, so daß der Kurvenverlauf der Ausgangssignale etwa dem Kurvenverlauf der Eingangssignale entspricht. Diese Kurvenverläufe sind so ausgebildet, daß sie nach einer gewissen Latenzzeit bis zu einem Maximum ansteigen und danach wieder abfallen. Dabei hängen die Steilheit des Anstieges und des Abfalls sowie die Verweilzeit der Kurve auf ihrem Maximum von der Art der Muskelstimulation ab. Werden die Muskeln mit einzelnen Impulsen in größeren zeitlichen Abständen stimuliert, dann steigt die Kurve relativ mäßig bis zum Maximum an und fällt sodann wieder mäßig ab. Wird dagegen mit vielen kurz aufeinander folgenden Impulse stimuliert, steigt die Kurve relativ steil an, verbleibt längere Zeit auf dem Maximum und fällt dann relativ steil wieder ab.

Aus diesen als Kraft-Zeit-Diagramm dargestellten Kurven ist weder die zeitliche Zuordnung der Amplituden deutlich erkennbar, noch gehen aus ihnen die Steigungs- bzw. Abfallkriterien signifikant hervor. Diese Informationen können aber für eine Auswertung dieser Kurvenverläufe wichtig sein.

Die Erfindung zielt darauf ab, diese Unsicherheiten der Auswertung zu verringern und insbesondere die Charakteristika der aufsteigenden und abfallenden Kurven-Äste sowie die zeitliche Zuordnung der Amplituden besser erfassen zu können. Außerdem zielt die Erfindung darauf ab, die Untersuchung von Muskelgewebe zu verbessern.

Dieses Ziel wird erfindungsgemäß durch den Gegenstand des Anspruchs 1 erreicht, also durch ein Gerät zum Untersuchen von mittels elektrischer Impulse stimulierter Muskeln oder Muskelgruppen, bei dem die Signale der mechanischen Reizantwort der Muskeln über mindestens einen Meßwertwandler, der mechanische Signale in elektrische Signale umwandelt und dessen Fühler hautextern auf den zu untersuchenden Muskeln aufliegt, an mindestens eine Darstellungs- und/oder Aufzeichnungsvorrichtung weiterleitbar sind, wobei der Meßwertwandler so ausgebildet ist, daß der Verlauf seiner elektrischen Ausgangssignale proportional der zeitlichen Ableitung (Differentiation) des Verlaufs seiner mechanischen Eingangssignale ist.

Die differenzierte Kurve zeigt nämlich den Wendepunkt, an dem im aufsteigenden bzw. abfallenden Ast der undifferenzierten Kurve eine Tendenzänderung eintritt, nunmehr als positives bzw. negatives Maximum an. Zusätzlich erscheinen die Extremwerte der undifferenzierten Kurve nunmehr als Schnittpunkte mit der Zeitachse und ermöglichen dadurch ein unmittelbares Ablesen des Zeitpunkts, an dem die Extremwerte auftreten.

Bei der Elektrostimulation von Muskeln ist es zwar bekannt (Veröffentlichung IEEE Transactions on Biomedical Engineering, Vol. 35, No 9, September 1988, Seite 758-763 insb. S. 762 KNAFLITZ), den Antwortsignalverlauf einer Differentiation zu unterziehen. Dabei handelt es sich jedoch nicht um eine mechanische Reizantwort, sondern um eine elektrische Reizantwort (Myoelectric signal), also um ein anderes Reizantwortsystem.

In den Unteransprüchen sind mehrere vorteilhafte Ausführungsformen des Gegenstandes des Anspruchs 1 dargestellt.

So gibt Anspruch 1 eine Tauchspulenanordnung an, mit der - ohne ein separates differenzierendes Übertragungsglied - ein differenziertes Ausgangssignal erzeugt wird, während nach Anspruch 2 ein separates differenzierendes Glied erforderlich ist. Besondere Ausführungsformen dieses differenzierenden Gliedes sind in Anspruch 4 bezeichnet.

Gemäß Anspruch 4 wird die Anwendung von besonderen Darstellungs- und Aufzeichnungsvorrichtungen vorgeschlagen.

Nach Anspruch 5 soll eine Unterteilung des Signalverlaufs in drei als Untersuchungskriterien wesentliche Hauptphasen in die Darstellung einbezogen werden. Dabei erfolgt eine Unterteilung des Signals in folgende Phasen:
- die Latenzzeit, in welcher die Stimulation beginnt, aber noch keine Reizantwort stattfindet (d.h. die Zeit vom Beginn des Impulses bzw. ersten Teilimpulses bis zum Beginn der Reaktion des Muskels),
- die aktive Phase, in welcher die Reizantwort stattfindet,
- und die Ausklingphase, in welcher nach Abschalten der Stimulation ein freies Ausschwingen stattfindet.

Gemäß Anspruch 1 erzeugt ein bevorzugtes Gerät elektrische Impulse, die jeweils aus mehreren Teilimpulsen bestehen. Somit wird ein Meßzyklus nicht wie bei anderen Ausgestaltungen der Erfindung durch einen einzelnen elektrischen Impuls eingeleitet, sondern durch einen Burst, d.h. eine Folge von zeitlich schnell aufeinanderfolgender, sehr kurzer Teilimpulse. Ein derartiger Burst ist aus folgendem Grund vorteilhaft: Infolge von kapazitiven Effekten im Gewebe, beispielsweise durch Ionenablagerungen an Membranen hervorgerufen, bildet das Gewebe für ein Gleichsignal einen hohen elektrischen Widerstand. Der Widerstand reduziert sich, wenn man ein Wechselsignal verwendet. Ein Burst wirkt wie ein Wechselsignal. Deshalb ist der elektrische Widerstand für einen Burst geringer. Infolgedessen kann man auch die angelegte elektrische Spannung reduzieren.

Die Anzahl derartiger einen Impuls bildenden Teilimpulse ist gemäß Anspruch 6 bevorzugt eine Zweierpotenz. Sie liegt insbesondere im Bereich von 4 bis 256 und beträgt beispielsweise 16. Die Verwendung von Zweierpotenzen ist vorteilhaft, da ein digitaler Steuerrechner zum Erzeugen der Teilimpulse sehr leicht mit Zweierpotenzen programmiert werden kann.

Ein erfindungsgemäßes Verfahren zur Leistungsfähigkeitsmessung gemäß Anspruch 7 findet Anwendung, um den physiologischen Zustandes von Muskeln bzw. Muskelgewebe festzustellen. Dieses Verfahren umfaßt die Schritte: Beaufschlagen des Muskelgewebes mit einem elektrischen Impuls, Aufnehmen eines Reizantwortsignal und Messen der Latenzzeit, der Dauer der aktiven Phase und/oder Dauer der Ausklingphase. Anstatt der Zeitdauern der einzelnen Phasen kann auch eine Kombination mehrerer Zeitdauern gemessen werden, bei-spielsweise die Dauer von Latenzzeit und aktiver Phase oder von aktiver Phase und Ausklingphase.

Das Messen bzw. Bestimmen der Zeitdauern anhand des aufgenommen Reizantwortsignals findet außerhalb des Körpers statt. Das Reizantwortsignal kann dazu entweder direkt vermessen oder zunächst zwischengespeichert und dann erst vermessen werden. Dabei vermißt vorzugsweise ein Computer das Reizantwortsignal. Dazu kann der Computer das aufgenommene Reizantwortsignal auch zunächst einer (weiteren) Signalverarbeitung unterziehen. Eine derartige Signalverarbeitung trägt dazu bei, charakteristische Eckdaten des Reizantwortsignals automatisch erfassen zu können, insbesondere die o.g. Latenszeit, die Dauern der aktiven Phase und der Ausklingphase, aber auch die Amplituden des Reizantwortsignals zu bestimmten Zeitpunkten.

Das Verfahren ist insbesondere geeignet zur Untersuchung der Leistungsfähigkeit und Belastbarkeit bzw. der Belastbarkeitsgrenzen eines Sportlers oder sonstigen Probanden bzw. deren Muskulatur. Das Verfahren kommt beispielsweise bei der Überwachung eines Trainingsprozesses eines Sportlers zur Anwendung, wobei sowohl eine Kurzzeitüberwachung, beispielsweise im Zeitraum von Minuten bis zu einigen Stunden, insbesondere während eines Trainingablaufes, als auch eine Langzeitüberwachung, beispielsweise im Zeitraum von Tagen bis zu etlichen Monaten, sinnvoll sein kann.

Außerdem findet das Verfahren bei der Überwachung und zum Nachweis von Rehabilitationsprozessen Anwendung. Da die Messungen Aufschluß über den Fortschritt derartiger Prozesse geben, läßt sich die Mitwirkung von Proband bzw. Patient sowie einer diesen anleitenden Person z.B. eines Arztes, überwachen. Anhand der Messungen - beispielweise beim Vergleich der Messungen vom Beginn und Ende eines Rehabilitationsprozesses - kann man die Qualität einer Rehabilitationseinrichtung beurteilen. Eine Qualitätseinstufung ist insbesondere interessant bei der Anerkennung förderungswürdiger Rehabilitationseinrichtungen, beispielsweise im Zusammenhang mit der Förderung durch Krankenkassen und Gesundheitsbehörden.

Das Verfahren ist ferner geeignet für Eignungsprüfungen und zur Tauglichkeitsfeststellung, beispielsweise bei Einstellungs- und Dienst(un)fähigkeitsuntersuchungen sowie Musterungen.

Bei dem erfindugsgemäßen Verfahren zur Leistungsfähigkeitsmesung der Muskulatur, d.h. einerseits einem Verfahren bei dem die elektrischen Impulse jeweils aus mehreren Teilimpulsen gebildet werden bzw. andererseits einem Verfahren bei dem zum Messen ein Fühler hautextern auf den zu untersuchenden Muskel aufgelegt wird, ergeben sich die im Zusammenhang mit dem erfindungsgemäßen Gerät erläuterten Vorteile.

Bei einem bevorzugten Verfahren wird das erfindungsgemäße Verfahren mehrmals, insbesondere in zeitlich sehr kurzen Abständen, hintereinander durchgeführt, d.h. das Muskelgewebe wird mehrfach mit einem elektrischen Impuls beaufschlagt, wobei die Reizantwort entsprechend oft wiederholt aufgenommen und Latenzzeit, Dauer der aktiven Phase und/oder Dauer der Ausklingphase und/oder eine Kombination dieser Zeiten ebenfalls entsprechend oft gemessen werden. Die so erhaltenen Meßwerte werden dann über die Anzahl der Wiederholungen gemittelt. Wenngleich sich in Versuchen gezeigt hat, daß die Meßwerte von zeitlich schnell aufeinanderfolgenden Messungen relativ konstant sind, erreicht man mit einer Mittelung einen Ausgleich verbleibender statistischer Schwankungen.

Die Anzahl derartiger Wiederholungen ist bevorzugt eine Zweierpotenz und liegt besonders bevorzugt im Bereich von 4 bis 256, besipielsweise 16. Die Verwendung von Zweierpotenzen ist vorteilhaft, da ein digitaler Steuerrechner sehr leicht mit Zweierpotenzen programmiert werden kann.

Gemäß Anspruch 13 wird das Untersuchungsverfahren schließlich mit einem erfindungsgemäßen Gerät durchgeführt.

Nachfolgend wird die Erfindung anhand bevorzugter Ausführungsbeispiele und der beigefügten schematischen Zeichnung näher beschrieben.

In der Zeichnung zeigen:
- Fig. 1: den zeitlichen Verlauf eines Eingangssignals eines Meßwertwandlers,
- Fig. 2: den zeitlichen Verlauf eines Ausgangssignals als Antwort des Meßwertwandlers auf ein Eingangssignal gemäß Fig. 1 und
- Fig. 3: eine vollständige Untersuchungsanlage mit einem bevorzugten Meßwertwandler-Typ.

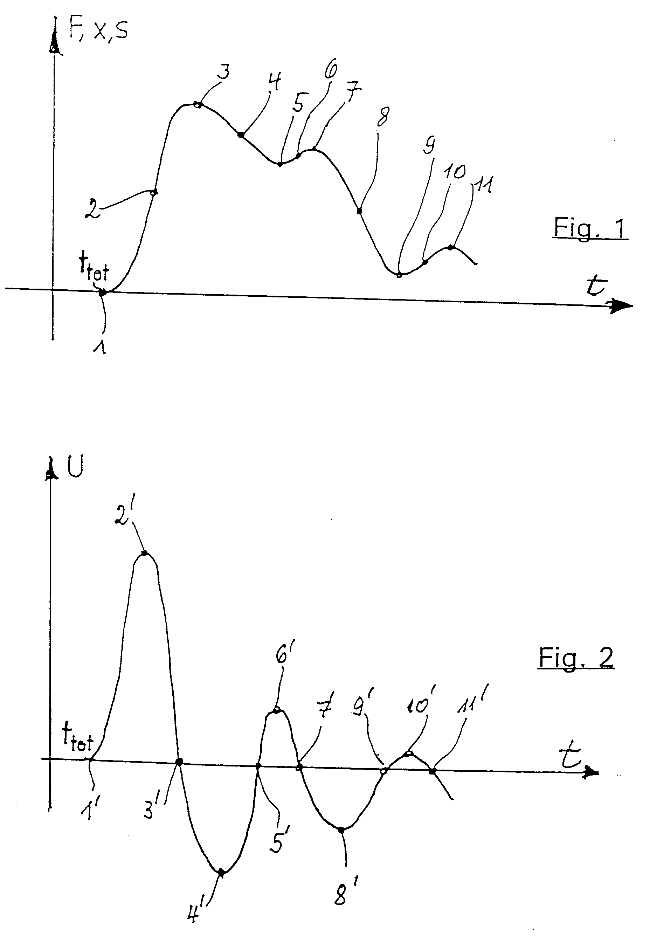

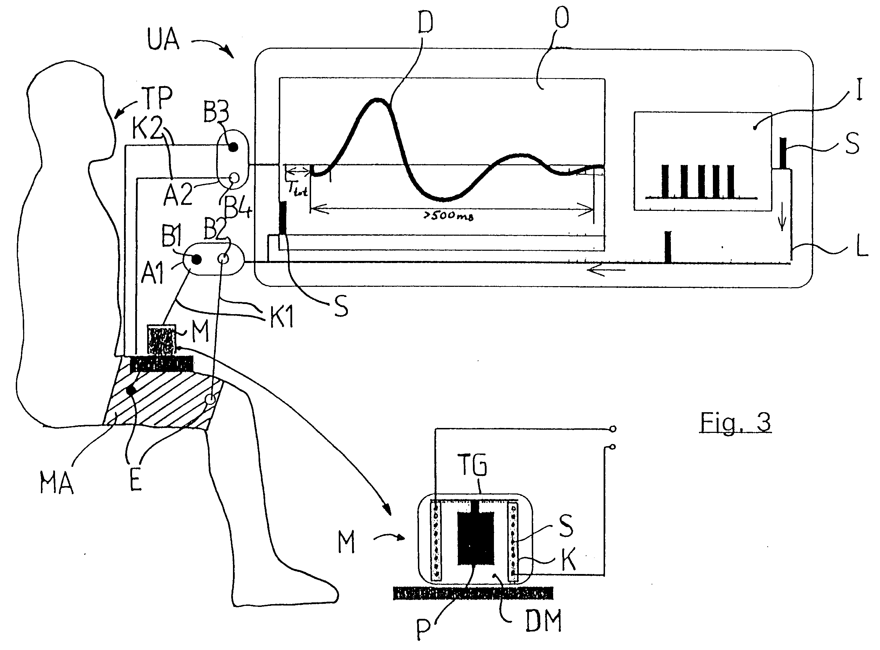

In der Zeichnung ist ein aus der Vielzahl der möglichen Signalverläufe ausgewählter Kurvenverlauf schematisiert dargestellt, und zwar in Fig. 1 ein Eingangssignal eines Meßwertwandlers und in Fig. 2 das zu diesem Eingangssignal korrespondierende Ausgangssignal des Meßwertwandlers.

Der Meßwertwandler wandelt ein mechanisches Signal, beispielsweise die Muskelkontraktion bzw. die Umfangsvergrößerung x eines Muskels oder die vom Muskel aufgebrachte Kraft F, in ein eletrisches Signal, beispielsweise in eine elektrische Spannung U, um. Der Meßwertwandler arbeitet als Schwingungsgeber nach Art einer Tauchspule. Diese Anordnung ist derart aufgebaut, daß ein Permanentmagnet in eine Schwingspule eintauchen kann. Dabei wird vom Muskel entweder der Permanentmagnet oder die Schwingspule bewegt, so daß es zu einer Relativbewegung zwischen Schwingspule und Permanentmagneten kommt. Diese Art der Messung ähnelt seismographischen Messungen, bei welchen durch Explosionen verursachte Schwingungen untersucht werden. Die Muskelaktivität kann mittels dieser Anordung nicht-invasiv gemessen werden.

Das Ausgangssignal des Meßwertwandlers ist proportional zur zeitlichen Ableitung des Eingangssignals. Das Ausgangssignal ändert sein Vorzeichen, wenn der Permanentmagnet und die Schwingspule ihre Bewegungsrichtung zueinander ändern. Eine beispielhafte Relativbewegung des Permanentmagneten zur Schwingspule, dargestellt durch die Auslenkung s der Schwingspule in Bezug auf den Permanentmagneten, ist in Fig. 1 gezeigt. Die Auslenkung s ist im wesentlichen proportional zur Muskelkontraktion bzw. der Umfangsvergrößerung x des Muskel und/oder der vom Muskel aufgebrachten Kraft F. Deshalb sind die Kraft F, die Muskelkontraktion bzw. Umfangsvergrößerung x und die Auslenkung s gemeinsam auf der Ordinatenachse in Fig. 1 aufgetragen.

Der korrespondierende Verlauf des Ausgangssignals, vgl. Fig. 2, kann beispielsweise auf einem getriggerten Speicheroszilloskop dargestellt und anschließend ausgewertet werden. Es kann aber auch ein Computer zur Darstellung und/oder Auswertung zum Einsatz kommen. Die charakteristischen Punkte sind in Fig. 1 mit den Bezugszeichen 1 bis 10 und in Fig. 2 mit den Bezugszeichen 1 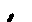 bis 10' bezeichnet.

Zur Überprüfung bzw. Untersuchung eines Muskels bzw. einer Muskelgruppe wird ein sehr kurzer elektrischer Impuls auf den Muskel bzw. die Muskelgruppe zum Zeitpunkt t=0 gegeben. Dieser Impuls führt nach einer Totzeit tₜₒₜ zu einer Muskelanregung, der sogenannten aktiven Phase. Die Impulsdauer ist auch gegenüber dieser Totzeit tₜₒₜ kurz, beispielsweise ist die Impulsdauer kleiner als 0,2 ms, während die Totzeit tₜₒₜ 7 bis 30 ms beträgt. Die aktive Phase liegt in Fig. 1 zwischen den Punkten 1 und 3 bzw. in Fig. 2 zwischen den Punkten 1' und 3'. Anschließend schwingt der Muskel in einer sogenannten Ausklingphase oder nichtaktiven Phase aus. Diese Ausklingphase wird in Fig. 1 durch die Punkte 3 bis 11 bzw. in Fig. 2 durch die Punkte 3' bis 11' beschrieben. Die Dauer der gesamten Muskelaktivität, insbesondere der meßbaren Muskelaktivität, - als Antwort auf einen Impuls - ist typischerweise größer als 500 ms.

Anhand des Meßsignals bzw. mehrerer Meßsignale kann/können insbesondere festgestellt werden:
- Veränderungen des Muskels, insbesondere auch bei zunehmender Ermüdung,
- der Zustand der Muskelzellen,
- aus der Latenzzeit bzw. Totzeit tₜₒₜ die Funktionalität der Muskelzelle, sowie biochemische Prozesse im Muskel,
- die Dynamik der Muskulatur und
- die Nervenmuskel-Leitungsfunktion.

Die Totzeit tₜₒₜ gibt ferner Aufschluß über die Membranpermeabilität des Muskelgewebes und die aktive Phase über die Menge der rekrutierten Muskelfasern und die Kontraktionseigenschaften des Muskels. Die abklingende bzw. nichtaktive Phase charakterisiert schließlich die elastisch-plastischen Eigenschaften, den Trainingszustand und den Grad der Ermüdung der Muskulatur und des Umgebungsgewebes. Diese Phase gibt auch Aufschluß über den Tonus (Spannungszustand) des Muskelgewebes.

Das Verhältnis der Amplituden der lokalen Maxima und/oder Minima in Fig. 2 charakterisiert den Dämpfungsgrad und somit die passiven Eigenschaften der Muskulatur. Dieses Verhältnis kann beispielsweise aus den Amplituden zu den Punkten 2' und 4', 2' und 6', 6' und 10', 4' und 8', usw. gebildet werden. Die Punkte 2', 6', 10' und die Punkte 4' und 8' werden jeweils von einer Exponentialfunktion eingehüllt. Der Dämpfungsgrad bestimmt auch den Verlauf dieser Exponentialfunktionen.

Das für die Untersuchung besonders aufschlußreiche Verhalten des Muskels vom Beginn der Stimulationsantwort bis zum Erreichen der ersten maximalen Reaktion, also der Bereich zwischen den Punkten 1 und 3 der Kurve in Fig. 1, zeigt sich in der Kurve in Fig. 2 als deutliche Zacke, deren Spitze durch den Punkt 2' gebildet ist, welche das Ansteigen und den Beginn des Nachlassens der ersten Reaktion verdeutlicht.

Ähnlich aufschlußreich sind auch die weiteren Zacken 6 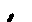 und 10 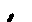 , weil sie das Ansteigen und Nachlassen der weiteren Reaktionen deutlich machen.

Die Punkte 3', 5', 7 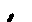 , 9' und 11 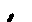 der unteren Kurve, die den Extremwerten bei der Muskelreaktion entsprechen, befinden sich sämtlich auf der Zeitachse und gestatten daher eine objektive Beurteilung hinsichtlich ihres Zeitwerts und hinsichtlich der Lage zueinander.

Insgesamt gibt die Auswertung der Punkte 1-11 bzw. 1'-11', insbesondere bezüglich der Latenzzeit bzw. Totzeit tₜₒₜ (charakterisiert durch den Punkt 1 bzw. 1') der aktiven Phase (charakterisiert durch die Punkte 1-3 bzw. 1'-3') und der abklingenden bzw. nichtaktiven Phase (charakterisiert durch die Punkte 3-11 bzw. 3'-11') Aufschluß über das neuro-muskuläre System der Trainingsperson.

Die Untersuchungsanlage UA nach Fig. 3 weist einen Impulsgenerator I und zwar einen programmierbaren Elektromyostimulator auf. Er ist insofern programmierbar, als die Höhe, d.h. die Stromstärke oder Spannung, die Dauer, der Abstand und/oder die Anzahl der Impulse von einer Bedienperson eingegeben werden können. Diese Werte können auch automatisch in Abhängigkeit eines Programms variiert werden. Der Impulsgenerator I wird von einer (nicht dargestellten) Stromquelle gespeist. Beispielsweise erzeugt er fortlaufend im zeitlichen Abstand elektrische Stromimpulse S von jeweils etwa 0,2 ms Dauer, die über eine Leitung L zu Elektroden E geleitet werden, die im hautexternen Kontakt z.B. mit dem Oberschenkelmuskel einer Trainingsperson TP (bzw. Probanden) stehen und in diesen die elektrischen Impulse einleiten.

Die Untersuchungsanlage UA weist einen Anschluß A1 mit zwei Buchsen B1, B2 auf. Diese Buchsen B1, B2 dienen zur Aufnahme von Steckern eines Kabels K1, das mit einer die Elektroden E aufweisenden Manschette MA verbunden ist. Die Manschette MA umschließt einen Muskel bzw. eine Muskelgruppe, und zwar gemäß Fig. 3 die Oberschenkelmuskulatur.

Die entsprechenden mechanischen Reizantwort-Signale, die sich im wesentlichen als normal (senkrecht) zur Muskeloberfläche gerichtete Vibrationen darstellen, weisen einen für den betreffenden Muskel charakteristischen Kurvenverlauf auf, der von einem mechanische Impulse in entsprechende elektrische Signale umwandelnden Meßwertwandler M aufgenommen und nach Differentierung einem Speicheroszilloskop O zur Auswertung zugeleitet wird. Der Meßwertwandler M weist ein Kabel K2 mit Steckern auf, welche mit Buchsen B3, B4 eines Anschlusses A2 der Untersuchungsanlage UA verbunden sind.

Bei dem vorliegenden Ausführungsbeispiel besteht der Meßwertwandler M aus einem elektromagnetischen Tauchspulen-Geber TG, auch Tauchmagnet-Geber genannt. Dieser weist eine feststehende, stromdurchflossene Spule S auf, die auf einem langen unmagnetischen Spulenkörper K aufgebracht ist. Mit der Spule wirkt ein stabförmiger Permanentmagnet P derart zusammen, daß er sich achsgleich mit dieser in deren Höhlung hin- und herbewegt, wobei diese Hin- und Herbewegungen den genannten Muskelvibrationen entsprechen. Zum Vermeiden bzw. zum Vermindern von zusätzlichen systemeigenen Schwingungen vollführt der Permanentmagnet P seine Bewegungen in einem Dämpfungsmedium DM, beispielsweise einem Öl oder einer anderen viskosen Masse, z.B. einem Gel.

Der Vorzug dieser Geber liegt vor allem darin, daß sie quantitativ große Meßsignale liefern, was vielfach eine Weiterverarbeitung der Signale auch ohne zusätzliche Verstärker ermöglicht.

Da es zum Wesen derartiger Tauchmagnet-Geber gehört, daß die Hin- und Herbewegungen des Permanentmagneten P in der Spule S eine entsprechende Differentialkurve der Eingangssignalkurve liefern, so werden auch im vorliegenden Fall die mechanischen Kurvenverläufe in entsprechende Differentialkurven D umgewandelt, die dann auf dem Speicheroszilloskop O abgebildet werden.

Aus diesen Differentialkurven D können dann für die Muskeltherapien, bei denen mit verschiedenartigen Muskelstimulationen gearbeitet wird, die wichtigen Daten entnommen werden, wie sie im einzelnen bereits in der Beschreibung zu Fig. 1 erläutert worden sind. Alternativ kann auch ein Tauchmagnet-Gebertyp eingesetzt werden, bei dem der Permanentmagnet stillsteht und die Spule die den Muskelvibrationen entsprechende Hin- und Herbewegung ausführt.

## Patentansprüche

1. Gerät (UA) zur Leistungsmessung von mittels elektrischer Impulse (S) stimulierter Muskeln oder Muskelgruppen, bei dem die Signale der mechanischen Reizantwort der Muskeln über mindestens einen Meßwertwandler (M; TG), der mechanische Signale in elektrische Signale umwandelt und dessen Fühler hautextern auf den zu untersuchenden Muskeln aufliegt, an mindestens eine Darstellungs- und/oder Aufzeichnungsvorrichtung (O) weiterleitbar sind,
dadurch gekennzeichnet,
daß der Meßwertwandler (M; TG) so ausgebildet ist,
daß der Verlauf (D) seiner elektrischen Ausgangssignale proportional der zeitlichen Ableitung (Differentation) des Verlaufs seiner mechanischen Eingangssignale ist, und daß der Meßwertwandler (M; TG) nach Art einer Tauchspule (S) mit einem in Längsrichtung zur Tauchspule (S) beweglichen Permanentmagneten ausgebildet ist, wobei entweder der Permanentmagnet (P) oder die Tauchspule (S) als Fühler für die Aufnahme der mechanischen Signale des Muskels dient und die Tauchspule (S) mit den ihr bei der Bewegung des Permanentmagneten (P) induzierten Strömen als Ausgangssignalgeber wirkt, wobei der elektrische Impuls (S) des Gerätes (UA) aus mehreren Teilimpulsen besteht.

2. Gerät (UA) nach Anspruch 1, dadurch gekennzeichnet, daß der Meßwertwandler (M; TG) ein erstes und ein zweites Glied aufweist, wobei
- das erste Glied mechanische Signale proportional in elektrische Signale umwandelt und
- das zweite Glied dem ersten Glied als Übertragungsglied mit differenzierendem Verhalten nachgeschaltet ist.

3. Gerät (UA) nach Anspruch 2, dadurch gekennzeichnet, daß das erste Glied als Magnetfeldgeber/Hall-Sensor, Dehnungsmeßstreifen oder piezoelektrische Folie ausgebildet ist.

4. Gerät (UA) nach einem der Ansprüche 1 bis 3, dadurch gekennzeichnet, daß die Darstellungsvorrichtung und die Aufzeichnungsvorrichtung ein getriggertes Speicheroszilloskop (O) und/oder einen Computer aufweisen.

5. Gerät (UA) nach einem der Ansprüche 1 bis 4, dadurch gekennzeichnet, daß die Darstellung und/oder Auswertung eine Unterteilung des Signalverlaufs in drei charakteristischen Phasen, nämlich
- die Latenzzeit, in welcher die Stimulation beginnt, aber noh keine Reizantwort stattfindet,
- die aktive Phase, in welcher die Reizantwort stattfindet, und
- die Ausklingphase, in welcher nach Abschalten der Stimulation ein freies Ausschwingen stattfindet,
berücksichtigt.

6. Gerät (UA), nach einem der Ansprüche 1 bis 5, dadurch gekennzeichnet, daß die Anzahl der einen Impuls (S) bildenden Teilimpulse eine Zweierpotenz ist, insbesondere eine Zweierpotenz im Bereich von 4 bis 256.

7. Verfahren zur Leistungsfähigkeitsmessung der Muskulatur mit den Schritten:
a) Beaufschlagen des Muskelgewebes mit einem elektrischen Impuls;
b) Aufnehmen eines mechanischen Reizantwortsignals; und
c) Messen der
i) Latenzzeit,
ii) Dauer der aktiven Phase und/oder
iii) Dauer der Ausklingphase und/oder
iv) einer Kombination dieser Zeiten, wobei der elektrische Impuls aus mehreren Teilimpulsen gebildet wird, und bei dem zum Messen
ein
Fühler hautextern auf den zu untersuchenden Muskel aufgelegt wird.

8. Verfahren nach Anspruch 7, bei dem die Verfahrensschritte a) bis c) mehrmals durchgeführt und die erhaltenen Meßwerte gemittelt werden.

9. Verfahren nach Anspruch 7 oder 8, bei dem ein Gerät nach einem der Ansprüche 1 bis 8 eingesetzt wird.
